# EUROPEAN PATENT SPECIFICATION

(11) **EP 4 272 651 B1**
(45) Date of publication and mention of the grant of the patent: **02.04.2025**
(21) Application number: 23170129.3
(22) Date of filing: 26.04.2023
(51) Int. Cl.: A61B 6/03, A61B 6/04, A61B 6/00

(54) **MEDICAL ANALYSIS APPARATUS**
MEDIZINISCHE ANALYSEVORRICHTUNG
APPAREIL D'ANALYSE MÉDICALE

(30) Priority: 04.05.2022 IT 202200009080
(43) Date of publication of application: 08.11.2023
(60) Divisional application: 25159508.8
(73) Proprietor: IMS Giotto S.p.A., 40037 Sasso Marconi (Bologna) (IT)
(72) Inventor: VIGNOLI, Paolo, SAN GIOVANNI IN PERSICETO (Bologna) (IT); BENASSI, Matteo, CASALECCHIO DI RENO (Bologna) (IT); MORSELLI, Massimo, SAN FELICE SUL PANARO (Modena) (IT)
(74) Representative: Milli, Simone

(56) References cited:
- WO-A1-2012/102435
- US-A1- 2017 172 530
- US-A1- 2021 128 087

## Description

This invention relates to a medical apparatus for X-ray analyses.

There are known medical X-ray apparatuses which comprise a source configured to emit X-rays and a detector configured to receive the X-rays emitted by the source.

Such apparatuses can be machines to perform, for example, mammography, tomosynthesis, breast CT, biopsies, CESM.

In order to perform the above-mentioned medical tests, the patient's breast is interposed between the X-ray source and the detector.

In this way, the breast may be passed through by the X-ray radiation which has been emitted by the source, so that the image of the breast is generated on the detector.

During the above-mentioned medical analyses the patient can be positioned vertically or in the prone position.

There are prior art apparatuses which are able to perform various types of medical analyses with the possibility for the patient to position him/herself in different positions.

Document US 2021/128087 A1 shows a system for multi-mode breast x-ray imaging, configured for a plurality of imaging protocols and modes, which comprises a compression arm assembly for compressing and immobilizing a breast for x-ray imaging, an x-ray tube assembly, and an x-ray image receptor. Document WO 2012/102435 concerns an X-ray mammography imaging device that includes a power transmission body and a mechanism for driving the same body, which allows reducing both the weight and dimensions of the rotating arm as well as the vibrations and noises produced.

Document US 2017/172530 relates to an integrated device for performing mammography, tomosynthesis, and stereotactic biopsy in seated, standing, and prone positions, where the X-ray source and detector plate are capable of moving at different angles relative to each other to facilitate image acquisition and the execution of stereotactic breast biopsies in seated, standing, and prone positions of the patient.

These apparatuses, as well as also their manufacture, are currently very complex and expensive.

Moreover, the prior art apparatuses require a very elaborate mechanical system which often translates into a heavy and bulky machine structure, in particular with regard to the portions of structure (for example, motors, reducers, transmission belts, movement arms) which allow the various necessary movements of the various components.

The need is felt in the trade for making apparatuses which are able to move the various components to have a compact structure, which is simple and with reduced costs.

The need is also felt to make apparatuses for medical X-ray analyses which allow a safe and effective movement of some portions of the apparatus.

An aim of the invention is to provide an apparatus for X-ray examinations which overcomes the above-mentioned drawbacks.

More specifically, an aim of the invention is to provide an apparatus for medical X-ray examinations which reduces the degree of complexity of the structure of the apparatus compared with the prior art.

Another aim of the X-ray apparatus according to the invention is to guarantee a high degree of safety and precision during the movements of portions of the structure of the apparatus during the examination step or preparation for the examination. The invention is set out in the appended set of claims.

The features of the invention are clearly described in the claims below and its advantages are more apparent from the detailed description which follows, with reference to the accompanying drawings which illustrate a preferred, non-limiting example embodiment of the invention and in which:

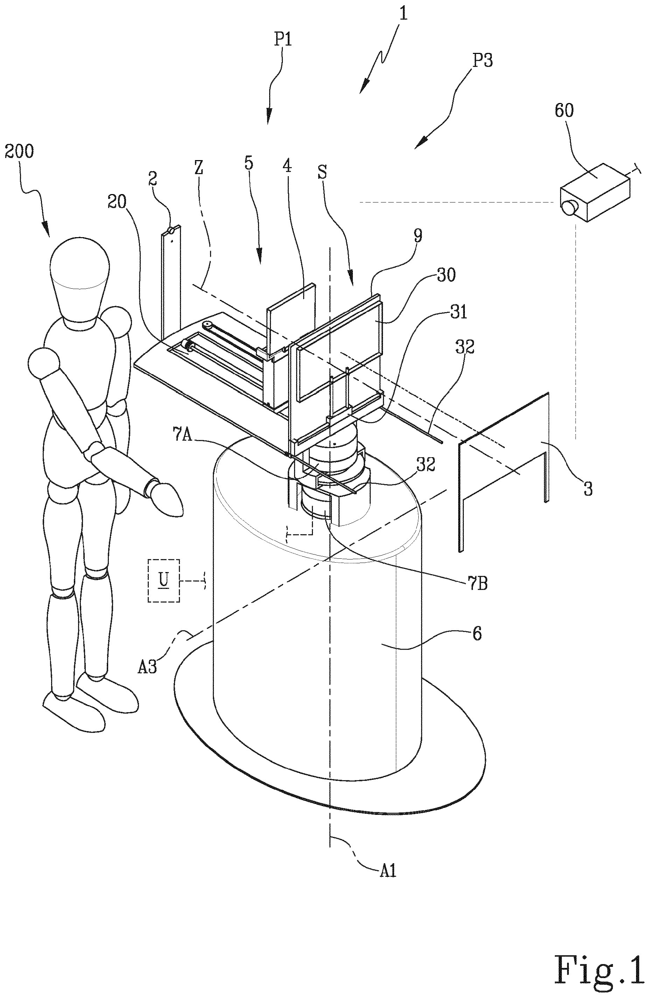

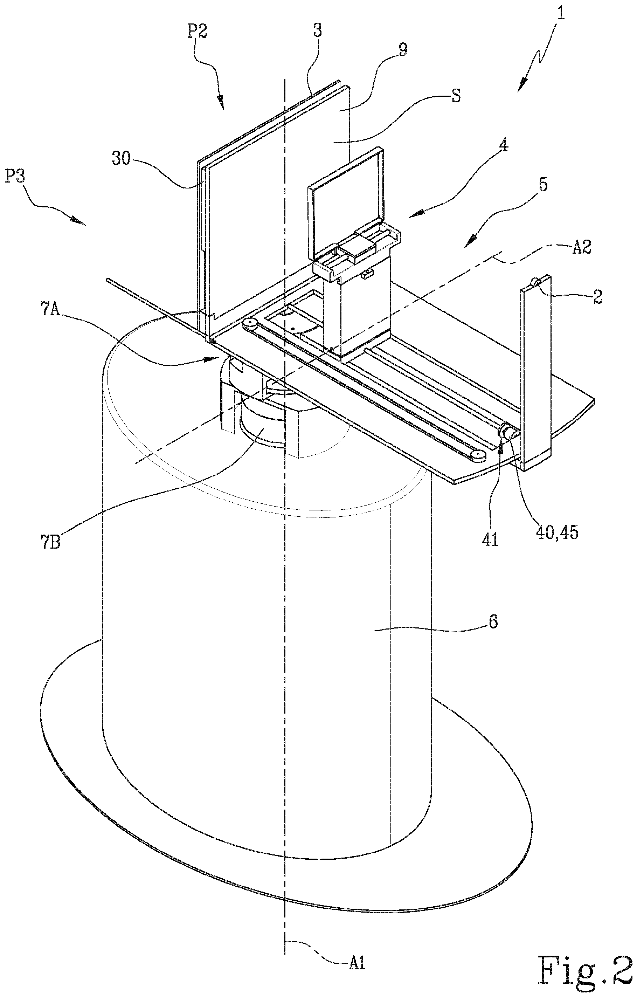

- Figures 1 and 2 are perspective views of two different operating configurations of an embodiment of the apparatus according to the invention;

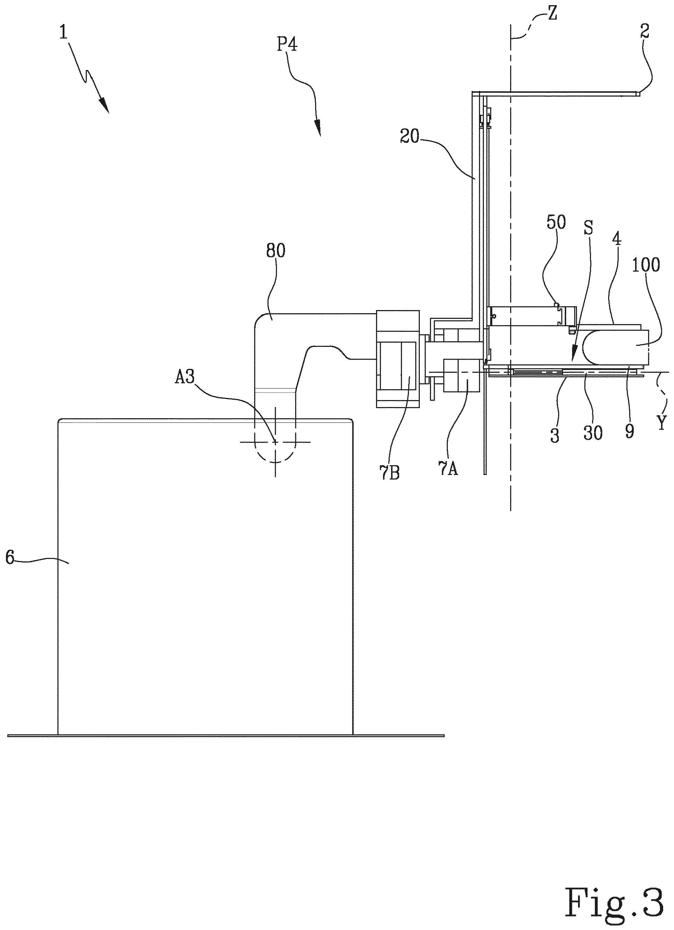

- Figure 3 is a side view of a representation of the apparatus according to the invention;

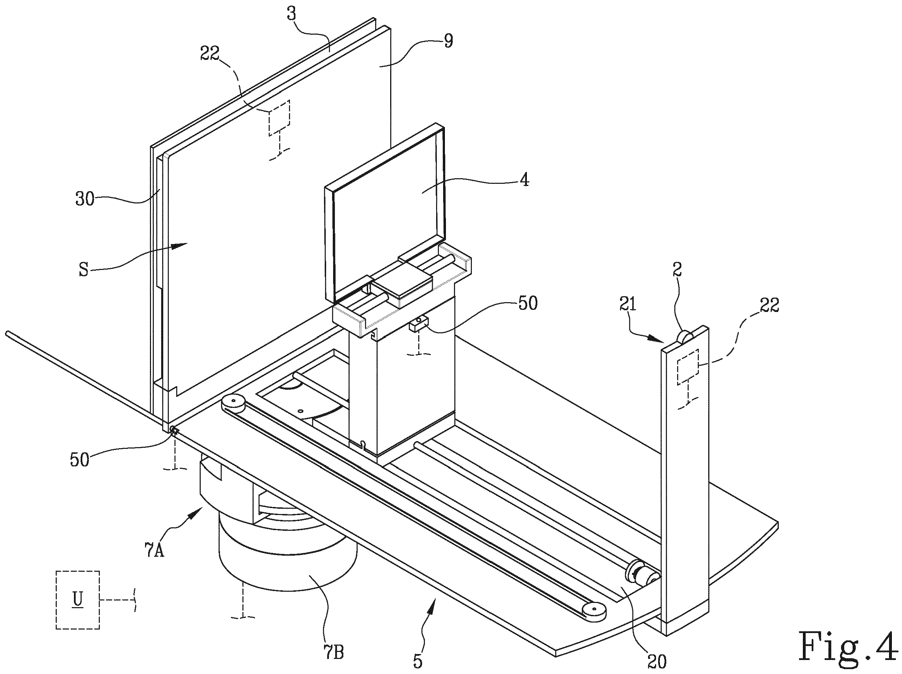

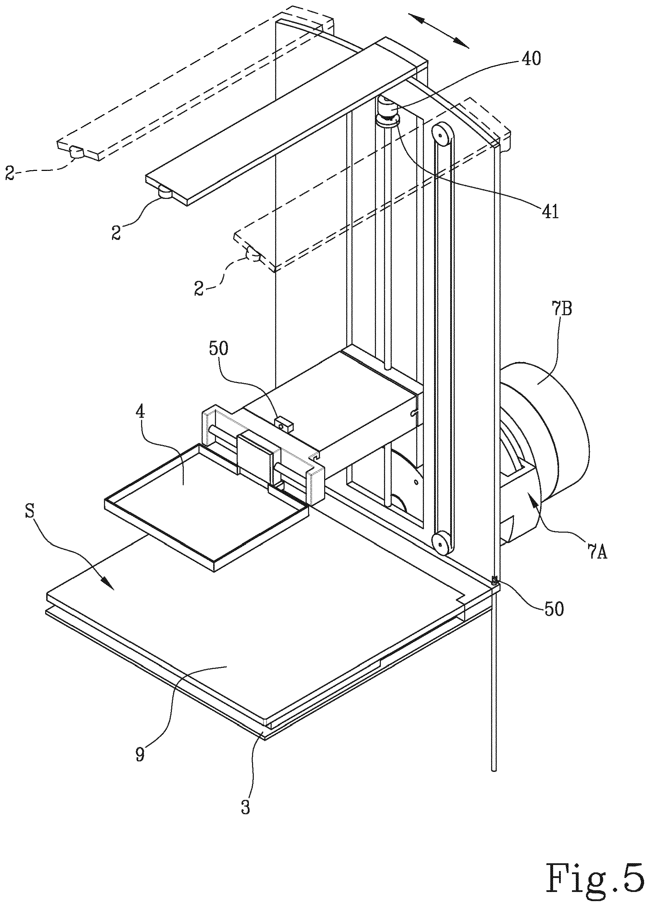

- Figures 4 and 5 are perspective views of details of the apparatus according to the invention;

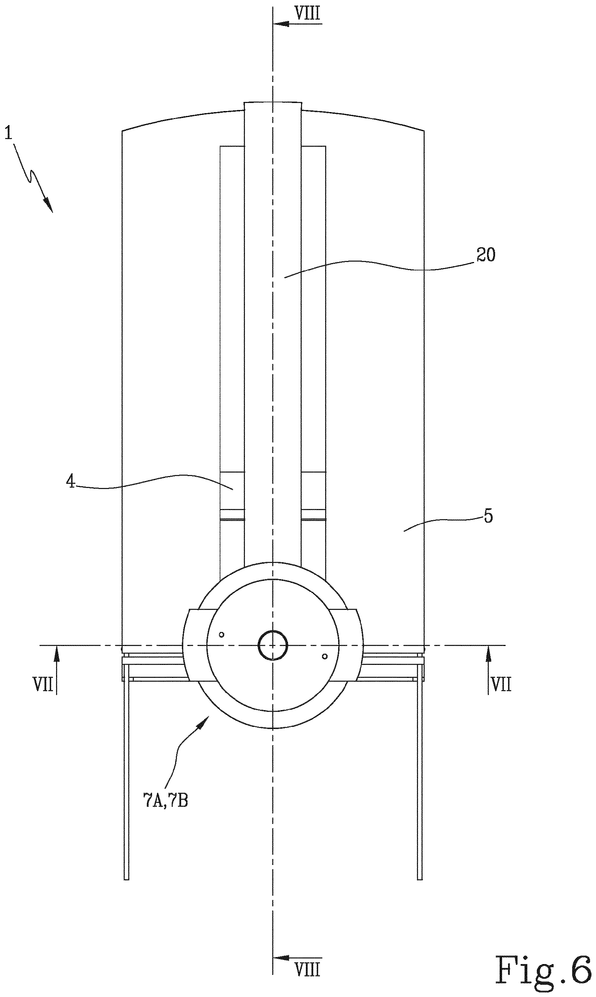

- Figure 6 is a rear view of a representation of a detail of the apparatus according to the invention;

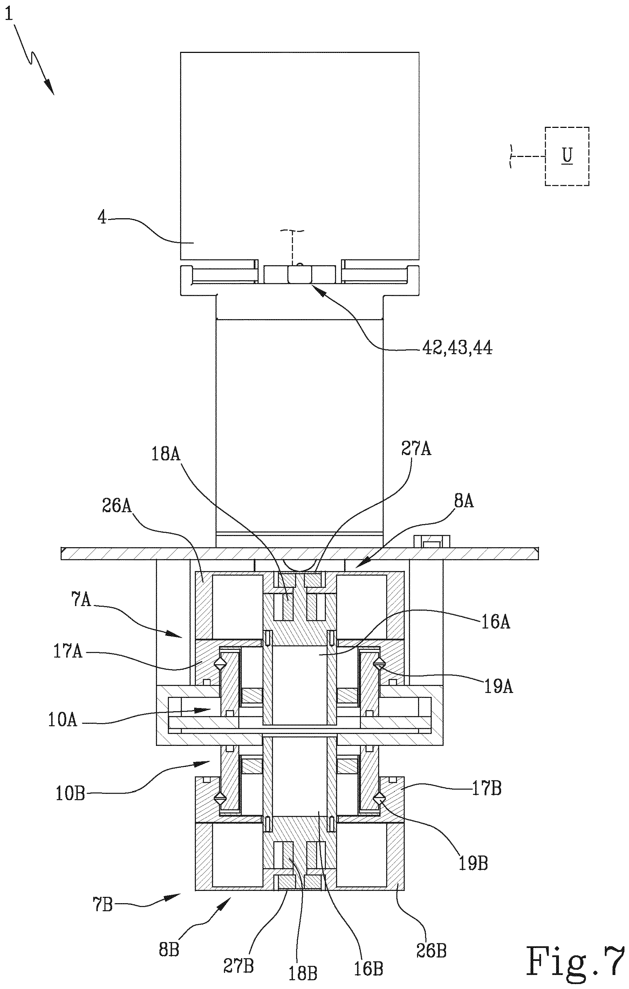

- Figure 7 is a view, partly in cross section, of Figure 6 along plane VII-VII;

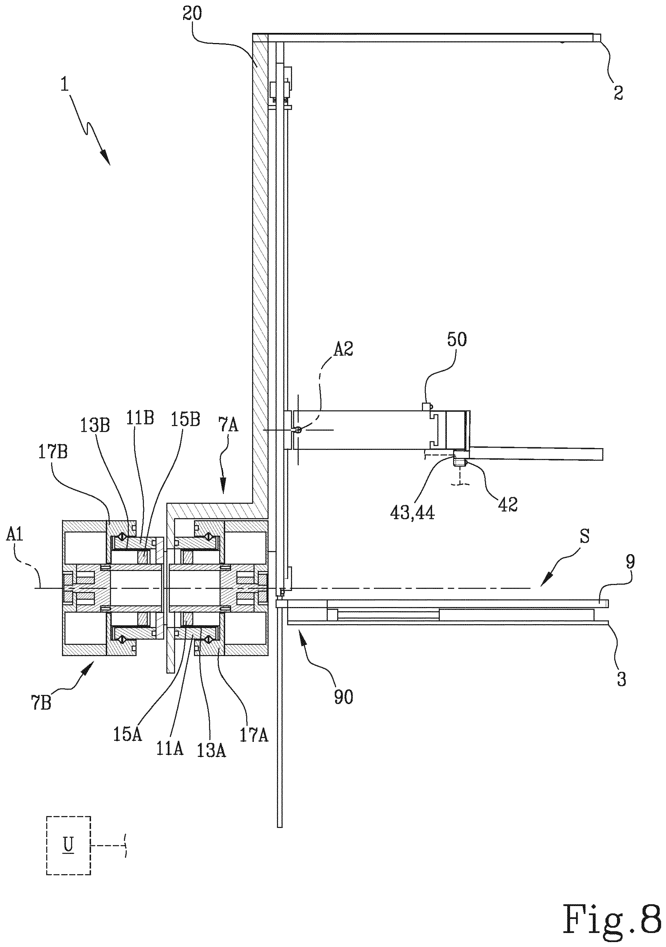

- Figure 8 is a view, partly in cross section, of Figure 6 along plane VIII-VIII;
- Figure 9 is a detail, shown in cross section, of a detail of Figure 6 along plane VIII-VIII.

It should be noted that all the drawings are schematic and are therefore not representative of the actual dimensions of the system.

With reference to the accompanying drawings, the numeral 1 denotes a medical apparatus for X-ray analysis.

According to the invention, the apparatus 1 comprises:
- at least one source 2 configured to emit X-rays;
- at least one X-ray detector 3 defining a detection plane S;
- an immobiliser 4 movable along a first direction Z perpendicular to the detection plane S;
- a supporting frame 5, supporting the at least one source 2, the at least one X-ray detector 3, the immobiliser 4;
- a base frame 6 movably supporting the supporting frame 5;
- a control unit U connected at least to the supporting frame 5, to the source 2 and to the X-ray detector 3 to receive a signal relating to the X-rays detected by the at least one X-ray detector 3 and configured to process said signal and derive an image.

The apparatus 1 comprises a first actuator 7A and a second actuator 7B. According to a preferred embodiment, the first actuator 7A and the second actuator 7B are coaxial gear motors.

The first actuator 7A is now described from a mechanical point of view. According to the invention, the first actuator 7A comprises a motor 8A.

According to the invention, the first actuator 7A comprises a transmission shaft 16A.

The motor 8A is configured to rotate the transmission shaft 16A about the first axis A1 of rotation.

According to the invention, the first actuator 7A comprises a locking device 18A acting on the transmission shaft 16A.

The first actuator 7A comprises a housing body 26A and the motor 8A, the transmission shaft 16A and the locking device 18A are positioned inside the housing body 26A.

The internal arrangement of the housing body 26A makes the structure of the first actuator 7A compact.

According to the invention, the first actuator 7A comprises a gear unit 10A. The gear unit 10A of the first actuator 7A comprises at least one rigid outer gear 11A with inner toothing 12A and a deformable inner gear 13A with outer toothing 14A.

The outer toothing 14A of the inner gear 13A is configured to engage with the inner toothing 12A of the rigid outer gear 11A, interfering mechanically with the rigid outer gear 11A for transmitting the rotational motion around the first axis A1 of rotation to the rigid outer gear 11A.

The gear unit 10A comprises an oval element 15A.

The expression "oval element" means a rigid, curved body which is non-circular in shape, having a major axis and a minor axis.

The oval element 15A is configured for connecting the transmission shaft 16A to the deformable inner gear 13A.

In other words, the oval element 15A is interposed between the transmission shaft 16A and the deformable inner gear 13A.

The oval element 15A is integral in rotation about the first axis A1 of rotation to the motor 8A.

In particular, the transmission shaft 16A is configured for transmitting to the oval element 15A the rotational motion about the first axis of rotation A1.

Preferably, the oval element 15A is positioned inside the inner gear 13A and is configured, in use, to apply a pressure in two zones (radially opposite each other) of the deformable inner gear 13A.

This pressure carries the two zones (radially opposite) of the deformable inner gear 13A in contact with two corresponding zones of the rigid outer gear 11A, engaging two portions of the outer toothing 14A of the deformable inner gear 13A with the corresponding portions of the inner toothing 12A of the rigid outer gear 11A.

According to an aspect of the invention, the number of teeth of the outer toothing 14A of the deformable inner gear 13A is different from (less than) the number of teeth of the inner toothing 12A of the rigid outer gear 11A. The first actuator 7A comprises bearings interposed between the oval element 15A and the deformable inner gear 13A.

These bearings allow the oval element 15A to rotate inside the deformable inner gear 13A without having a rotation integral with each other.

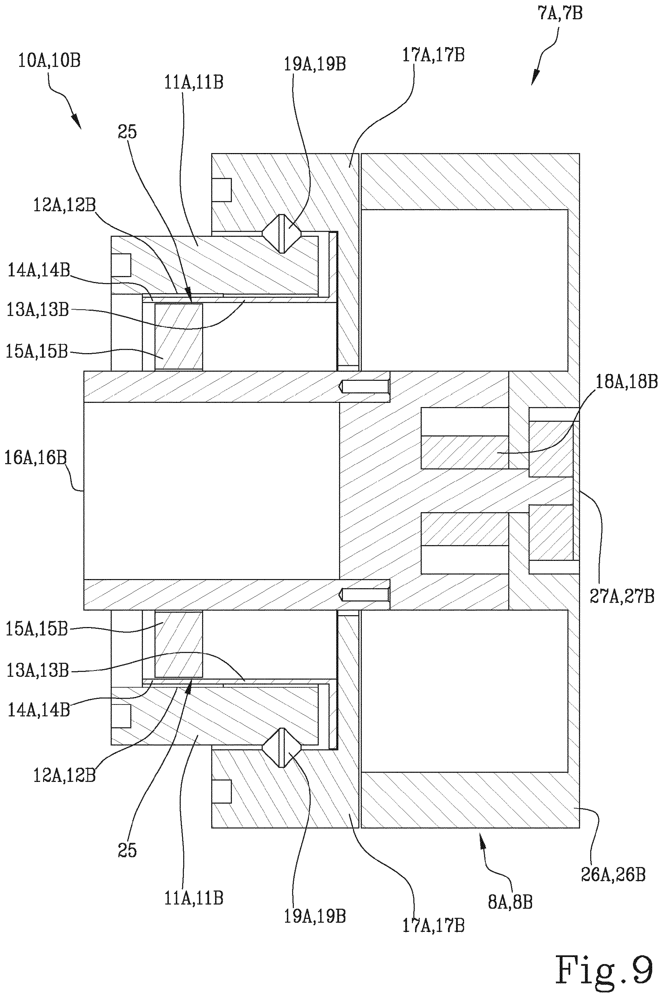

In Figure 9 the numeral 25 denotes the annular zone in which the bearings are positioned.

In short, the oval element 15A rotates inside the deformable inner gear 13A deforming it.

It should be noted that this rotation in the presence of bearings, together with the difference in the number of teeth between the outer toothing 14A of the deformable inner gear 13A and the inner toothing 12A of the rigid outer gear 11A, allows, in use, the oval element 15A to continuously modify the portions of the outer toothing 14A and the inner toothing 12A mutually engaged.

More specifically, in use, each tooth of the outer toothing 14A of the deformable inner gear 13A engages and disengages with a first dip of the inner toothing 12A of the rigid outer gear 11A and then engages and disengages with another, second, dip of the inner toothing 12A.

This process is repeated continuously and the distance between two consecutive dips for engaging a same tooth depends on the difference in the number of teeth between the outer toothing 14A of the deformable inner gear 13A and the inner toothing 12A of the rigid outer gear 11A. This continuous engagement and disengagement between the teeth 14A, 12A transmits the rotational motion about the first axis A1 of rotation of the oval element 15A to the rigid outer gear 11A.

In particular, the number of teeth less than the outer toothing 14A of the deformable inner gear 13A with respect to the inner toothing 12A of the rigid outer gear 11A allows the rigid outer gear 11A to rotate with an angular speed less than that of the oval element 15A.

Advantageously, by adjusting the ratio between the number of teeth of the outer toothing 14A of the deformable inner gear 13A and the inner toothing 12A of the rigid outer gear 11A it is possible to control the angular speed of rotation of the rigid outer gear 11A.

Advantageously, this mechanical mechanism between outer toothing 14A of the deformable inner gear 13A and inner toothing 12A of the rigid outer gear 11A allows a zero-clearance gear motor to be obtained, that is to say, a gear motor in which there is an extremely small clearance between teeth of one toothing (14A, 12A) and corresponding engaged dips of the other toothing (12A, 14A).

Advantageously, having an extremely reduced clearance between the toothing (14A, 12A) being coupled does not result in their wear. According to an aspect of the invention, the first actuator 7A comprises a fixed portion 17A, that is to say, which cannot be rotated, directly or indirectly, by the motor 8A.

The first actuator 7A comprises a plurality of bearings 19A interposed between the rigid outer gearing 11A and the fixed portion 17A and configured to facilitate the sliding of one relative to the other.

Preferably, the bearings 19A are crossed roller bearings.

In other words, when the rigid outer gear 11A rotates about the first axis A1 of rotation, it slides relative to the fixed portion 17A which, on the other hand, does not rotate relative to the axis A1 by direct or indirect action of the motor 8A.

According to an aspect of the invention, the first actuator 7A comprises a position sensor 27A.

Preferably, the position sensor 27A is an encoder.

The position sensor 27A is positioned inside the housing body 26A.

The position sensor 27A is coupled to the transmission shaft 16A.

The position sensor 27A is positioned inside the housing body 26A. According to an embodiment, the gear unit 10A, the position sensor 27A, the locking device 18A, the housing body 26A and elements of the motor 8A (for example, rotor and stator) are made in a single cast block. Advantageously, making these components in a single cast block makes manufacturing rapid and less complex.

According to an aspect of the invention, the first actuator 7A comprises a control card.

The control card is connected to the control unit U and the control unit U is configured to control the control card.

The transmission shaft 16A, driven by the motor 8A, is configured for transmitting to the gear unit 10A the rotational motion about the first axis A1 of rotation.

According to a preferred embodiment, the transmission shaft 16A is connected to the oval element 15A for transmitting to the oval element 15A the rotational motion about the axis A1 of rotation.

Preferably, the locking device 18A is configured for locking said transmission shaft 16A with respect to the rotation.

In short, the locking device 18A makes the transmission shaft 16A stationary and consequently locks relative to rotation everything that is connected to the transmission shaft 16A.

The locking device 18A locks the gear unit 10A relative to the rotation about the first axis A1 of rotation.

In particular, the locking device 18A locks the rotation of the oval element 15A.

Advantageously, the mechanics described allow the first actuator 7A to be coaxial relative to the first axis A1 of rotation.

Advantageously, the mechanics described allow the first actuator 7A to be compact.

Advantageously, these mechanics allows a silent movement. Advantageously, these mechanics allows a movement without vibrations. Advantageously, these mechanics allows a high reduction ratio between the angular speeds of the transmission shaft 16A and the rigid outer gear 11A.

It should be noted that in order for the inner gear 13A to be deformable it necessarily has a large number of small teeth and therefore a high reduction ratio.

It should be noted that the mechanics of the second actuator 7B, which for brevity of description will not be described in detail, are similar to that described for the first actuator 7A, bearing in mind that:
a motor 8B corresponds to the motor 8A;
a gear unit 10B corresponds to the gear unit 10A;
a rigid outer gear 11B corresponds to the rigid outer gear 11A;
an inner toothing 12B of the rigid outer gear 11B corresponds to the inner toothing 12A of the rigid outer gear 11A;
a deformable inner gear 13B corresponds to the deformable inner gear 13A;
an outer toothing 14B of the deformable inner gear 13B corresponds to the outer toothing 14A of the deformable inner gear 13A;
an oval element 15B corresponds to the oval element 15A;
a transmission shaft 16B corresponds to the transmission shaft 16A;
a fixed portion 17B corresponds to the fixed portion 17A;
a locking device 18B corresponds to the locking device 18A;
a plurality of bearings 19B corresponds to the plurality of bearings 19A;
a housing body 26B corresponds to the housing body 26A;
a position sensor 27B corresponds to the position sensor 27A.

The second actuator 7B also comprises a similar operation to, and brings the same advantages as, that described above for the first actuator 7A. According to the invention, the first actuator 7A configured to rotate the X-ray source 2 about a first axis of rotation A1 parallel to the detection plane S.

In other words, the first actuator 7A is configured to move the X-ray source 2 in different positions along an arc of a circle with a centre on the axis A1 of rotation for acquiring stereotaxic and tomographic images.

Preferably, the distance between the first axis A1 of rotation and the detector 3 is less than the distance between the first axis A1 of rotation and the source 2.

Preferably, the distance between the first axis A1 of rotation and the detector 3 is less than or equal to 150 mm, even more preferably less than or equal to 100 mm.

According to a preferred embodiment, the apparatus 1 comprises an arm 20 for supporting the X-ray source 2.

The first actuator 7A is configured to rotate the supporting arm 20 about the first axis A1 of rotation.

The locking device 18A of the first actuator 7A, configured to act directly on the transmission shaft 16A, allows the supporting arm 20 or the X-ray source 2 to be kept locked to the rotation.

It should be noted that, advantageously, the locking device 18A which acts on the transmission shaft 16A may be of reduced size, in particular with respect to the dimensions and the mass of the supporting arm 20 and/or of the X-ray source 2.

In this way, the overall dimensions of the apparatus 1 are extremely reduced.

According to an embodiment, the locking device 18A has a diameter of between 2 cm and 10 cm, in particular between 3 and 6 cm, more preferably between 3 cm and 4 cm.

In effect, the high reduction ratio guaranteed by the mechanics of the first actuator 7A allows the use of a simple locking device 18A of reduced size. In effect, this reduction ratio guarantees the possibility of acting directly on the transmission shaft 16A, and opposing efficiently the rotation of the supporting arm 20 or of the X-ray source 2.

A locking device 18A of these dimensions guarantees compactness and a smaller size than the first actuator 7A, guaranteeing at the same time the required efficiency, and it is also more economical than more complex or large devices.

Advantageously, the possibility of positioning the X-ray source 2 in different positions along the arc makes it possible to acquire stereotaxic or tomographic images of a breast 100 and/or of the immobiliser 4. Advantageously, the stereotaxic or tomographic images allow reliable information to be obtained even deep inside the breast 100.

According to the invention, the second actuator 7B is configured to rotate the supporting frame 5 about the above-mentioned first axis of rotation A1 between a first operating position P1 and a second operating position P2. This rotation makes it possible to perform a diagnostic test in the first operating position P1, in the second operating position P2 or in a position between them.

In particular, considering a breast 100 interposed between the X-ray source 2 and the detector 3, while keeping the position of the patient fixed, this rotation allows the apparatus 1 to irradiate the breast 100 from different angles.

The possibility of emitting X-rays on the breast 100 at different angles allows an operator 200 to set the optimum operating position for the diagnostic test to be performed.

It should be noted, more specifically, that the X-ray source 2 is supported by the supporting frame 5 and therefore when the second actuator 7B rotates about the first axis of rotation A1 the supporting frame 5 the X-ray source 2 also rotates in an integral fashion.

The first actuator 7A, on the other hand, allows the X-ray source 2 to be rotated about the first A1 axis of rotation, without simultaneously rotating the supporting frame 5 and the other elements supported by it.

According to an aspect of the invention, the first actuator 7A and the second actuator 7B are mechanically coupled to each other.

According to a preferred embodiment, the rigid outer gear 11B of the second actuator 7B is connected to the supporting frame 5.

The locking device 18B of the second actuator 7B, configured for acting directly on the transmission shaft 16B, makes it possible to keep the supporting frame 5 locked to rotation. It should be noted that, advantageously, the locking device 18B which acts on the transmission shaft 16B may be of reduced dimension, in particular with respect to the dimensions and mass of the supporting frame 5.

In this way, the overall dimensions of the apparatus 1 are extremely reduced.

According to an embodiment, the locking device 18B has a diameter of between 2 cm and 10 cm, in particular between 3 and 6 cm, more preferably between 3 cm and 4 cm.

In effect, the high reduction ratio guaranteed by the mechanics of the second actuator 7B allows the use of a simple locking device 18B of reduced size. In fact, this reduction ratio guarantees the possibility of acting directly on the transmission shaft 16B, and efficiently opposing the rotation of the supporting frame 5.

A locking device 18B of these dimensions guarantees compactness and a smaller size than the second actuator 7B, guaranteeing at the same time the required efficiency, and it is also more economical than more complex or large devices.

The supporting frame 5 is connected to the motor 8A of the first actuator 7A.

In essence, the second actuator 7B rotates the supporting frame 5 about the first axis A1 of rotation, which rotates in an integral fashion the motor 8A of the first actuator 7A. Consequently, the first actuator 7A is rotated in an integral fashion.

The first actuator 7A is connected to the X-ray source 2. The rotation of the first actuator 7A about the first axis A1 of rotation, as a result of the rotation of the supporting frame 5, rotates in an integral fashion the X-ray source 2 about the first axis A1 of rotation.

Considering the preferred embodiment having the supporting arm 20 of the X-ray source 2, the first actuator 7A is connected to the supporting arm 20. The rotation of the first actuator 7A about the first axis A1 of rotation rotates the supporting arm 20 about the first axis A1 of rotation. According to a preferred embodiment, the rigid outer gear 11A of the first actuator 7A is connected to the supporting arm 20 (or X-ray source 2). Preferably, these connections between the rigid inner gear 11B and the supporting frame 5 and/or between the supporting frame 5 and the motor 8A of the first actuator 7A and/or between the rigid outer gear 11A and the supporting arm 20 (or X-ray source 2) are connections using pin mechanisms.

The mechanical features of the first and second actuators (7A, 7B) described above advantageously allow a compact structure of the apparatus 1.

In particular, the motor (8A, 8B), the transmission shaft (16A, 16B) and the locking device (18A, 18B) positioned inside the housing body (26A, 26B), together with the structure of the first and second actuators (7A, 7B) and the connections described between the first actuator 7A, the second actuator 7B, the supporting frame 5 and the X-ray source 2, make the apparatus 1 very compact, keeping the apparatus 1 optimised in terms of its functions.

The compact apparatus 1 allows the operator 200 to move freely around the patient and the apparatus itself during a diagnostic examination or in preparation for it.

It should be noted that the mechanics and geometry described for the first and the second actuators (7A, 7B), and for their coupling, allow a largely negligible (substantially zero) clearance to be obtained between the rotation of the X-ray source 2 and that of the X-ray detector 3. This negligible clearance (substantially zero) allows the X-ray source 2 and the X-ray detector 3 to be locked to each other, maintaining the relative position in a precise and efficient manner using exclusively the locking device (18A, 18B) acting directly on the transmission shaft (16A, 16B). According to this invention, the control unit U is configured to control the first actuator 7A and the second actuator 7B.

The control unit U is configured to control the control card present in each actuator (7A, 7B).

The control unit U is configured to control the drive of the motor (8A, 8B) of each actuator (7A, 7B).

Preferably, the control unit U comprises an interface through which the operator 200 is able to control the first actuator 7A and the second actuator 7B.

The interface is equipped with commands, more specifically control pushbuttons.

According to an aspect of the invention, the apparatus 1 comprises a supporting body 9 for the breast 100 positioned between the detection plane S and the immobiliser 4.

According to a preferred embodiment, the supporting body 9 is flat. According to an embodiment, the supporting body 9 of the breast 100 is removably connected to the supporting frame 5.

The supporting body 9 is movable along the first direction Z.

Preferably, the supporting body 9 is movable along the direction Z in a range of between 5 cm and 25 cm, more preferably between 12 cm and 18 cm, even more preferably between 14.5 cm and 15.5 cm.

The X-ray detector 3 is movable along the first direction Z.

This mobility of the X-ray detector 3 along the first direction Z allows the distance between the axis A1 of rotation and the detector 3 to be increased with respect to the preferred embodiment described above. This allows the apparatus 1 to be configured to also perform, for example, BCBCT examinations wherein the detector 3 must be able to rotate around the breast 100 without striking it.

Preferably, the apparatus 1 comprises guides 32 and the supporting body 9 and the X-ray detector 3 are movable along the first direction Z along the guides 32.

According to an aspect of the invention, the apparatus 1 comprises a grille 30 for removing diffuse radiation, interposed between the supporting body 9 of the breast 100 and the X-ray detector 3.

The apparatus 1 comprises an actuator 31 for moving said grille 30 at least along a second direction Y perpendicular to the first direction Z. Preferably, the actuator 31 for moving the grille 30 is positioned on the opposite side of the source 2 with respect to the X-ray detector 3.

More specifically, considering the position of the detector 3 along the first direction Z as the "height 0", the X-ray source 2 is positioned at a positive height and the movement actuator 31 of the grille 30 is positioned at a negative height along the first direction Z.

Advantageously, the position described for the movement actuator 31 facilitates the operator 200 during the manoeuvres for setting up the immobiliser 4 (in particular during compression of the breast 100). According to an aspect of the invention, the grille 30 is integral with the X-ray detector 3 in the movements along the first direction Z, that is to say, it moves towards and away from the X-ray source 2 together with the detector 3.

In other words, the grille 30, the X-ray detector 3 and the supporting surface 9 of the breast 100 are movable together along the first direction Z.

According to an aspect, the apparatus 1 comprises a further actuator 90 configured for moving the X-ray grille 30 and/or the X-ray detector 3 and/or the supporting surface 9 of the breast 100 along the first direction Z. Preferably, the further actuator 90 is positioned on the opposite side of the source 2 with respect to the X-ray detector 3.

Advantageously, the position described for the further actuator 90 facilitates the operator 200 during the manoeuvres for preparing the diagnostic examination and approach of the patient to the apparatus 1. According to an aspect of the invention, the apparatus 1 comprises an actuator 40 for moving the immobiliser 4 configured to move the immobiliser 4 along the first direction Z.

According to an aspect, the movement actuator 40 of the immobiliser 4 comprises a coaxial movement motor 45.

According to another aspect, the movement actuator 40 of the immobiliser 4 comprises a belt and a worm screw which are coaxial with respect to the movement actuator 40 of the immobiliser 4.

According to an aspect, the control unit U is configured to control the movement actuator 40 of the immobiliser 4.

Preferably, the interface of the control unit U is configured to control the movement actuator 40 of the immobiliser 4.

In other words, the operator 200 can move the immobiliser 4 along the first direction Z by means of the interface of the control unit U.

The movement actuator 40 of the immobiliser 4 comprises a coupling and release mechanism 41 movable between a coupling position and a release position.

This coupling and release mechanism 41 is configured for selectively setting up a mode of automatic movement of the immobiliser 4 along the first direction Z, in the coupling position, or a mode for manual movement of the immobiliser 4 along the first direction Z, in the release position.

In other words, the coupling/release mechanism 41 controls the mode of movement of the immobiliser 4 allowing it to be both actuated directly by means of the control unit U and moved automatically or it to be moved only manually.

In short, the coupling/release mechanism 41 selectively determines whether the movement motor 45 is operatively active, in the coupling position, or is not operatively active, in the release position.

Preferably, the coupling/release mechanism 41 is a friction mechanism. According to an embodiment, the coupling/release mechanism 41 is of the magnetic type.

Advantageously, this coupling and release mechanism 41 allows the immobiliser 4 to be moved also in the presence of any electro-mechanical faults to the apparatus 1 or in a power failure. In particular, it allows the patient to be released and disconnected from the machine even under these conditions.

According to an aspect of the invention, the immobiliser 4 comprises at least one detection sensor 42 configured to detect a presence of the breast 100 or a part of the patient's body.

The detection sensor 42 is, for example, a temperature sensor.

According to an aspect, the control unit U is connected to the detection sensor 42 and is configured to receive information from the detection sensor 42 upon the detection of the presence of the breast 100 or a part of the patient's body. In this case, the control unit U is configured to slow down or lock the movement of the immobiliser 4.

Advantageously, detecting a presence of the breast 100 or of a part of the body of the patient during movement of the immobiliser 4 allows any impacts of the immobiliser 4 with the patient to be predicted and/or avoided.

According to an embodiment, the immobiliser 4 comprises at least a first force sensor 43 and a second force sensor 44.

The first force sensor 43 is configured to detect a contact with the breast 100 and the second force sensor 44 is configured to detect a force applied on the immobiliser 4.

The control unit U is connected to the first force sensor 43 and to the second force sensor 44 and is configured to receive information relating to the data detected by the force sensors (43, 44).

The control unit U is configured to slow down the speed of movement of the immobiliser 4 upon receiving the information from the first force sensor 43 relative to a contact of the immobiliser 4 with the breast 100. According to an aspect, the first force sensor 43 is a load cell.

According to an aspect, the second force sensor 44 is a load cell.

The control unit U is configured for deriving the force exerted by the immobiliser 4 on the breast 100 during compression from the information received from the second force sensor 44 relating to the detection of the force exerted on the immobiliser 4.

According to an embodiment, the immobiliser 4 is movable in rotation about a second axis A2 of rotation parallel to the detection plane S.

In other words, the immobiliser 4 can be inclined relative to the second axis A2 of rotation.

Advantageously, inclining the immobiliser 4 relative to the second axis A2 of rotation allows adaptation to the breast 100 in a more comfortable way for the patient and more efficient in locking the breast 100.

According to an aspect of the invention, the apparatus 1 comprises a plurality of monitoring sensors 50 configured for detecting the presence of an operator 200 and/or of the patient and/or of a component of the apparatus 1 in a predetermined monitoring area.

The monitoring sensors 50 are, for example, position or temperature sensors.

According to an aspect, the control unit U is connected to the monitoring sensors 50 and is configured to receive, by means of the monitoring sensors 50, information regarding the presence of an operator 200 and/or of the patient and/or of a component of the apparatus 1.

The control unit U is configured to slow down the speed of movement of the immobiliser 4 upon receiving the information from the monitoring sensors 50 relative to the presence of an operator 200 and/or of the patient and/or of a component of the apparatus 1.

Advantageously, the presence of the monitoring sensors 50 makes it possible to monitor predetermined surveillance areas, in particular in the proximity of the apparatus 1, during a diagnostic examination.

Monitoring these areas makes it possible to limit impacts, interference and other unforeseen events due to the presence of an operator 200 and/or of the patient and/or a component of the apparatus 1 in said areas. According to an embodiment, the apparatus 1 comprises a viewing system 60 configured to capture images of the immobiliser 4 and/or of the supporting body 9 of the breast 100 and/or of the breast 100 and/or of the patient and/or of the operator 200, to identify the position or the physical characteristics of one or more of said immobiliser 4, supporting body 9 of the breast 100, breast 100, patient, operator 200, with respect to a predetermined reference system.

Advantageously, in this way, it is possible to obtain information useful for operation of the apparatus 1, through the viewing system 60 itself, in particular with regard to the position of some elements of the apparatus 1 or of the breast 100 of the patient.

Preferably, the viewing system 60 comprises one or more video cameras, preferably of the stereo type.

The viewing system 60 may also perform the function of anti-collision checking, since the control unit U may be configured to allow a check of the relative positioning of the various components of the apparatus 1 to identify any abnormal positions which can correspond to a collision condition.

Generally speaking, it should be noted that the information regarding the positions of the breast 100 and/or lesions in the breast 100 and/or components of the apparatus 1 makes it possible to use all the possible configurations of the apparatus 1 described in the invention in order to optimise the diagnostic examination.

In a preferred embodiment, the supporting frame 5, the first actuator 7A and the second actuator 7B can be inclined, with respect to the base frame 6, by means of a rotation about a third axis of horizontal rotation A3 between a first operating position P3 and a second operating position P4 to allow a diagnostic examination to be performed in the first operating position P3 or in the second operating position P4.

According to an aspect, the apparatus 1 comprises an arm 80 configured for connecting the base frame 6 to the second actuator 7B and, therefore, as described above, to the first actuator 7A and to the supporting frame 5. The arm 80 is movable in rotation about the third axis A3 of rotation. Advantageously, the compact structure described for the first and second actuators (7A, 7B) allows a simpler and lighter structure of the apparatus 1 to be obtained.

A simpler and lighter structure of the apparatus 1, in particular with regard to the unit comprising the first actuator 7A, second actuator 7B and supporting frame 5, facilitates the movement of the arm 80 in rotation about the third axis A3 of rotation.

Advantageously, allowing a diagnostic examination to be performed in the first operating position P3 or in the second operating position P4 allows the apparatus 1 to perform different types of diagnostic examinations in different positions of the patient depending on the optimum configuration planned for the diagnostic examination.

Advantageously, the apparatus 1 according to this invention, thanks to its high degree of simplicity and compactness, allows an operator 200 a high level of freedom of movement and operation during, and in preparation for, a diagnostic examination.

According to an aspect of the invention, the apparatus 1 comprises a collimation device 21, configured to collimate the radiation beam emitted by the source 2.

According to a further aspect, the apparatus 1 comprises at least one control sensor 22 configured to detect a variation of the position of the focus of the beam of radiation emitted by the source 2 relative to the detector 3.

The control sensor 22 is, for example, an inclinometer.

The control sensor 22 is configured for sending a signal to the control unit U relative to this variation.

The control unit U is configured to control the collimation device 21 as a function of said signal sent by the control sensor 22 so as to adjust the collimation of the radiation beam.

According to a preferred embodiment, the apparatus 1 comprises a control sensor 22 positioned on the X-ray source 2 and a control sensor 22 positioned on the X-ray detector 3.

These sensors are configured to detect and measure the variation of the angular position of the source 2 relative to the X-ray detector 3.

The control unit U is configured to control the collimation device 21 as a function of the variation so as to adjust the collimation of the radiation beam.

It should be noted, for example, that the movements described for the apparatus 1 and its components might cause the above-mentioned variation of the angular position of the source 2 relative to the X-ray detector 3.

Advantageously, the apparatus 1 is able to optimise the collimation of the beam in each operating position of the apparatus 1.

Advantageously, the control sensors 22, together with the collimation device 21 and the control unit U, allow the collimation of the X-ray beam to be optimised by keeping the apparatus light, simple, compact and by limiting the costs.

## Claims

1. A medical apparatus (1) for X-ray analysis, comprising:
- at least one source (2) configured to emit X-rays,
- at least one X-ray detector (3) defining a detection plane (S),
- an immobiliser (4) movable along a first direction (Z) perpendicular to the detection plane (S),
- a supporting frame (5), supporting the at least one source (2), the at least one X-ray detector (3), the immobiliser (4),
- a base frame (6) movably supporting the supporting frame (5);
- a control unit (U) connected at least to the supporting frame (5), to the source (2) and to the X-ray detector (3) to receive a signal relating to the X-rays detected by the at least one X-ray detector (3) and configured to process said signal and derive an image;
- a first actuator (7A) configured to rotate the X-ray source (2) about a first axis of rotation (A1) parallel to the detection plane (S);
- a second actuator (7B) configured to rotate the supporting frame (5) about the above-mentioned first axis of rotation (A1) between a first operating position (P1) and a second operating position (P2) to allow a diagnostic examination to be performed in the first operating position (P1) or in the second operating position (P2);
the first actuator (7A) and the second actuator (7B) each comprising:
- a motor (8A, 8B);
- a transmission shaft (16A, 16B), operatively connected to the motor (8A, 8B) in order to be rotated;
- a locking device (18A, 18B) acting on said transmission shaft (16A, 16B);
- a housing body (26A, 26B);
the motor (8A, 8B), the transmission shaft (16A, 16B) and the locking device (18A, 18B) being positioned inside the housing body (26A, 26B), the control unit (U) being configured to control the first actuator (7A) and the second actuator (7B);
the apparatus (1) being **characterised in that** the first actuator (7A) and the second actuator (7B) each comprise a gear unit (10A, 10B) comprising:
- a rigid outer gear (11A, 11B) with inner toothing (12A, 12B),
- a deformable inner gear (13A, 13B) with outer toothing (14A, 14B) configured to engage with the inner toothing (12A, 12B) of the outer gear (11A, 11B), mechanically interfering with the rigid outer gear (11A, 11B) for transmitting the rotational motion to the rigid outer gear (11A, 11B),
- and an oval element (15A, 15B) configured for connecting the transmission shaft (16A, 16B) to the deformable inner gear (13A, 13B), the oval element (15A, 15B) being integral in rotation with the transmission shaft (16A, 16B).

2. The apparatus (1) according to the preceding claim, wherein each motor (8A, 8B) is configured to rotate the respective transmission shaft (16A, 16B) about the first axis of rotation(A1).

3. The apparatus (1) according to the preceding claim, wherein the first actuator (7A) and the second actuator (7B) each comprise a position sensor (27A, 27B), positioned inside the housing body (26A, 26B), and a control card.

4. The apparatus (1) according to any one of the preceding claims, wherein the first actuator (7A) and the second actuator (7B) are gear motors coaxial with respect to the first axis of rotation (A1).

5. The apparatus (1) according to any one of the preceding claims, wherein the first actuator (7A) and the second actuator (7B) are mechanically coupled.

6. The apparatus (1) according to any one of the preceding claims, wherein the first actuator (7A) is supported by the supporting frame (5) and is configured to rotate about the first axis of rotation (A1) in an integral fashion with respect to the supporting frame (5).

7. The apparatus (1) according to any one of the preceding claims, comprising a supporting surface (9) of the breast (100) and wherein the X-ray detector (3) and the supporting surface (9) of the breast (100) are movable along said first direction (Z).

8. The apparatus (1) according to any one of the preceding claims, comprising an actuator (40) for moving the immobiliser (4) configured for moving the immobiliser (4) along the first direction (Z), the actuator (40) for moving the immobiliser (4) comprising a coupling and release mechanism (41), movable between a coupling position and a release position, configured for selectively setting up a mode of automatic movement of the immobiliser (4) along the first direction (Z), in the coupling position, or manually moving the immobiliser (4) along the first direction (Z), in the release position.

9. The apparatus (1) according to any one of the preceding claims, wherein the immobiliser (4) comprises at least one detection sensor (42) configured to detect a presence of the breast (100) or a part of the patient's body.

10. The apparatus (1) according to any one of the preceding claims, wherein the immobiliser (4) comprises at least a first force sensor (43) and a second force sensor (44), the first force sensor (43) being configured to detect a contact with the breast (100) and the second force sensor (44) being configured to detect a force applied on the immobiliser (4).

11. The apparatus (1) according to any one of the preceding claims, wherein the immobiliser (4) is movable in rotation about a second axis of rotation (A2) parallel to the detection plane (S).

12. The apparatus (1) according to any one of the preceding claims, comprising a plurality of monitoring sensors (50) configured for detecting the presence of an operator (200) and/or of the patient and/or of a component of the apparatus (1) in a predetermined monitoring area.

13. The apparatus (1) according to any one of the preceding claims, comprising a viewing system (60) configured to capture images of the immobiliser (4) and/or of the supporting surface (9) of the breast (100) and/or of the breast (100) and/or of the patient and/or of the operator (200), to identify the position or the physical characteristics of one or more of said immobiliser (4), supporting surface (9) of the breast (100), breast (100), patient, operator (200), with respect to a predetermined reference system.

14. The apparatus (1) according to any one of the preceding claims, wherein the supporting frame (5), the first actuator (7A) and the second actuator (7B) can be inclined, with respect to the base frame (6), by means of a rotation about a third axis of horizontal rotation (A3) between a first operating position (P3) and a second operating position (P4) to allow a diagnostic examination to be performed in the first operating position (P3) or in the second operating position (P4).

## Patentansprüche

1. Medizinische Vorrichtung (1) zur Röntgenanalyse, umfassend:
- mindestens eine Quelle (2), die zum Aussenden von Röntgenstrahlen ausgelegt ist,
- mindestens einen Röntgendetektor (3), der eine Detektionsebene (S) definiert,
- eine Halteeinrichtung (4), die entlang einer ersten Richtung (Z) senkrecht zur Detektionsebene (S) bewegbar ist,
- einen Tragrahmen (5), der die mindestens eine Quelle (2), den mindestens einen Röntgendetektor (3) und die Halteeinrichtung (4) trägt,
- einen Grundrahmen (6), der den Tragrahmen (5) beweglich trägt;
- eine Steuereinheit (U), die zumindest mit dem Tragrahmen (5), der Quelle (2) und dem Röntgendetektor (3) verbunden ist, um ein Signal in Bezug auf die von dem mindestens einen Röntgendetektor (3) detektierte Röntgenstrahlung zu empfangen und ausgelegt ist, um dieses Signal zu verarbeiten und ein Bild abzuleiten;
- einen ersten Aktor (7A), der dazu ausgelegt ist, die Röntgenquelle (2) um eine erste Drehachse (A1) parallel zur Detektionsebene (S) zu drehen;
- einen zweiten Aktor (7B), der ausgelegt ist, um den Tragrahmen (5) um die oben genannte erste Drehachse (A1) zwischen einer ersten Betriebsposition (P1) und einer zweiten Betriebsposition (P2) zu drehen, um zu ermöglichen, dass eine diagnostische Untersuchung in der ersten Betriebsposition (P1) oder in der zweiten Betriebsposition (P2) durchgeführt wird;
wobei der erste Aktor (7A) und der zweite Aktor (7B) jeweils umfassen:
- einen Motor (8A, 8B);
- eine Übertragungswelle (16A, 16B), die mit dem Motor (8A, 8B) betriebswirksam verbunden ist, um gedreht zu werden;
- eine Sperreinrichtung (18A, 18B), die auf die Übertragungswelle (16A, 16B) wirkt;
- einen Gehäusekörper (26A, 26B);
wobei der Motor (8A, 8B), die Übertragungswelle (16A, 16B) und die Sperreinrichtung (18A, 18B) innerhalb des Gehäusekörpers (26A, 26B) positioniert sind,
wobei die Steuereinheit (U) ausgelegt ist, um den ersten Aktor (7A) und den zweiten Aktor (7B) zu steuern; wobei die Vorrichtung (1) **dadurch gekennzeichnet ist, dass** der erste Aktor (7A) und der zweite Aktor (7B) jeweils eine Getriebeeinheit (10A, 10B) umfassen, umfassend:
- ein starres Außenzahnrad (11A, 11B) mit Innenverzahnung (12A, 12B),
- ein verformbares Innenzahnrad (13A, 13B) mit einer Außenverzahnung (14A, 14B), das so ausgelegt ist, dass es mit der Innenverzahnung (12A, 12B) des Außenzahnrads (11A, 11B) in Eingriff steht und mechanisch mit dem starren Außenzahnrad (11A, 11B) interferiert, um die Drehbewegung auf das starre Außenzahnrad (11A, 11B) zu übertragen,
- und ein ovales Element (15A, 15B), das zum Verbinden der Übertragungswelle (16A, 16B) mit dem verformbaren Innenzahnrad (13A, 13B) ausgelegt ist, wobei das ovale Element (15A, 15B) drehfest mit der Übertragungswelle (16A, 16B) verbunden ist.

2. Vorrichtung (1) nach dem vorhergehenden Anspruch, wobei ein jeder Motor (8A, 8B) dazu ausgelegt ist, die jeweilige Übertragungswelle (16A, 16B) um die erste Drehachse (A1) zu drehen.

3. Vorrichtung (1) nach dem vorhergehenden Anspruch, wobei der erste Aktor (7A) und der zweite Aktor (7B) jeweils einen Positionssensor (27A, 27B), der innerhalb des Gehäusekörpers (26A, 26B) positioniert ist, und eine Steuerkarte umfassen.

4. Vorrichtung (1) nach einem der vorhergehenden Ansprüche, wobei der erste Aktor (7A) und der zweite Aktor (7B) Getriebemotoren sind, die in Bezug auf die erste Drehachse (A1) koaxial sind.

5. Vorrichtung (1) nach einem der vorhergehenden Ansprüche, wobei der erste Aktor (7A) und der zweite Aktor (7B) mechanisch gekoppelt sind.

6. Vorrichtung (1) nach einem der vorhergehenden Ansprüche, wobei der erste Aktor (7A) von dem Tragrahmen (5) getragen wird und dazu ausgelegt ist, sich integral in Bezug auf den Tragrahmen (5) um die erste Drehachse (A1) zu drehen.

7. Vorrichtung (1) nach einem der vorhergehenden Ansprüche, umfassend eine Tragfläche (9) der Brust (100) und wobei der Röntgendetektor (3) und die Tragfläche (9) der Brust (100) entlang der ersten Richtung (Z) bewegbar sind.

8. Vorrichtung (1) nach einem der vorhergehenden Ansprüche, umfassend einen Aktor (40) zum Bewegen der Halteeinrichtung (4), der zum Bewegen der Halteeinrichtung (4) entlang der ersten Richtung (Z) ausgelegt ist, wobei der Aktor (40) zum Bewegen der Halteeinrichtung (4) einen Kopplungs- und Freigabemechanismus (41) umfasst, der zwischen einer Kopplungsposition und einer Freigabeposition bewegbar ist, der zum selektiven Einrichten eines Modus der automatischen Bewegung der Halteeinrichtung (4) entlang der ersten Richtung (Z) in der Kopplungsposition oder zum manuellen Bewegen der Halteeinrichtung (4) entlang der ersten Richtung (Z) in der Freigabeposition ausgelegt ist.

9. Vorrichtung (1) nach einem der vorhergehenden Ansprüche, wobei die Halteeinrichtung (4) mindestens einen Detektionssensor (42) umfasst, der ausgelegt ist, um eine Anwesenheit der Brust (100) oder eines Teils des Körpers der Patientin zu detektieren.

10. Vorrichtung (1) nach einem der vorhergehenden Ansprüche, wobei die Halteeinrichtung (4) mindestens einen ersten Kraftsensor (43) und einen zweiten Kraftsensor (44) umfasst, wobei der erste Kraftsensor (43) dazu ausgelegt ist, einen Kontakt mit der Brust (100) zu detektieren, und der zweite Kraftsensor (44) dazu ausgelegt ist, eine auf die Halteeinrichtung (4) ausgeübte Kraft zu detektieren.

11. Vorrichtung (1) nach einem der vorhergehenden Ansprüche, wobei die Halteeinrichtung (4) in Drehung um eine zweite Drehachse (A2) parallel zur Detektionsebene (S) bewegbar ist.

12. Vorrichtung (1) nach einem der vorhergehenden Ansprüche, umfassend eine Vielzahl von Überwachungssensoren (50), die zum Detektieren der Anwesenheit eines/einer Bedieners/Bedienerin (200) und/oder der Patientin und/oder einer Komponente der Vorrichtung (1) in einem vorbestimmten Überwachungsbereich ausgelegt sind.

13. Vorrichtung (1) nach einem der vorhergehenden Ansprüche, umfassend ein Betrachtungssystem (60), das ausgelegt ist, um Bilder der Halteeinrichtung (4) und/oder der Tragfläche (9) der Brust (100) und/oder der Brust (100) und/oder der Patientin und/oder des/der Bedieners/Bedienerin (200) zu detektieren, um die Position oder die physikalischen Eigenschaften von einer oder mehreren der Halteeinrichtung (4), Tragfläche (9) der Brust (100), Brust (100), Patientin, Bediener/Bedienerin (200) in Bezug auf ein vorbestimmtes Bezugssystem zu identifizieren.

14. Vorrichtung (1) nach einem der vorhergehenden Ansprüche, wobei der Tragrahmen (5), der erste Aktor (7A) und der zweite Aktor (7B) in Bezug auf den Grundrahmen (6) durch eine Drehung um eine dritte horizontale Drehachse (A3) zwischen einer ersten Betriebsposition (P3) und einer zweiten Betriebsposition (P4) geneigt werden können, um zu ermöglichen, dass eine diagnostische Untersuchung in der ersten Betriebsposition (P3) oder in der zweiten Betriebsposition (P4) durchgeführt wird.

## Revendications

1. Appareil médical (1) d'analyse par rayons X, comprenant :
- au moins une source (2) configurée pour émettre des rayons X,
- au moins un détecteur de rayons X (3) définissant un plan de détection (S),
- un dispositif d'immobilisation (4) mobile le long d'une première direction (Z) perpendiculaire au plan de détection (S),
- un cadre de support (5), supportant l'au moins une source (2), l'au moins un détecteur de rayons X (3), le dispositif d'immobilisation (4),
- un cadre de base (6) supportant de manière mobile le cadre de support (5) ;
- une unité de commande (U) reliée au moins au cadre de support (5), à la source (2) et au détecteur de rayons X (3) pour recevoir un signal relatif aux rayons X détectés par l'au moins un détecteur de rayons X (3) et configurée pour traiter ledit signal et déduire une image ;
- un premier actionneur (7A) configuré pour faire tourner la source de rayons X (2) autour d'un premier axe de rotation (A1) parallèle au plan de détection (S) ;
- un second actionneur (7B) configuré pour faire tourner le cadre de support (5) autour du premier axe de rotation (A1) susmentionné entre une première position de fonctionnement (P1) et une seconde position de fonctionnement (P2) pour permettre la réalisation d'un examen diagnostique dans la première position de fonctionnement (P1) ou dans la seconde position de fonctionnement (P2) ;
le premier actionneur (7A) et le second actionneur (7B) comprenant chacun :
- un moteur (8A, 8B) ;
- un arbre de transmission (16A, 16B), relié de manière fonctionnelle au moteur (8A, 8B) afin d'être mis en rotation ;
- un dispositif de verrouillage (18A, 18B) agissant sur ledit arbre de transmission (16A, 16B) ;
- un corps de logement (26A, 26B) ;
le moteur (8A, 8B), l'arbre de transmission (16A, 16B) et le dispositif de verrouillage (18A, 18B) étant positionnés à l'intérieur du corps de logement (26A, 26B),
l'unité de commande (U) étant configurée pour commander le premier actionneur (7A) et le second actionneur (7B) ; l'appareil (1) étant **caractérisé en ce que** le premier actionneur (7A) et le second actionneur (7B) comprennent chacun une unité d'engrenages (10A, 10B) comprenant :
- un engrenage extérieur rigide (11A, 11B) doté d'une denture intérieure (12A, 12B),
- un engrenage intérieur déformable (13A, 13B) doté d'une denture extérieure (14A, 14B) configurée pour se mettre en prise avec la denture intérieure (12A, 12B) de l'engrenage extérieur (11A, 11B), interférant mécaniquement avec l'engrenage extérieur rigide (11A, 11B) pour transmettre le mouvement de rotation à l'engrenage extérieur rigide (11A, 11B),
- et un élément ovale (15A, 15B) configuré pour relier l'arbre de transmission (16A, 16B) à l'engrenage intérieur déformable (13A, 13B), l'élément ovale (15A, 15B) étant solidaire en rotation de l'arbre de transmission (16A, 16B).

2. Appareil (1) selon la revendication précédente, dans lequel chaque moteur (8A, 8B) est configuré pour faire tourner l'arbre de transmission (16A, 16B) respectif autour du premier axe de rotation (A1).

3. Appareil (1) selon la revendication précédente, dans lequel le premier actionneur (7A) et le second actionneur (7B) comprennent chacun un capteur de position (27A, 27B), positionné à l'intérieur du corps de logement (26A, 26B), et une carte de commande.

4. Appareil (1) selon l'une quelconque des revendications précédentes, dans lequel le premier actionneur (7A) et le second actionneur (7B) sont des motoréducteurs coaxiaux par rapport au premier axe de rotation (A1).

5. Appareil (1) selon l'une quelconque des revendications précédentes, dans lequel le premier actionneur (7A) et le second actionneur (7B) sont couplés mécaniquement.

6. Appareil (1) selon l'une quelconque des revendications précédentes, dans lequel le premier actionneur (7A) est supporté par le cadre de support (5) et est configuré pour tourner autour du premier axe de rotation (A1) de façon solidaire par rapport au cadre de support (5).

7. Appareil (1) selon l'une quelconque des revendications précédentes, comprenant une surface de support (9) du sein (100) et dans lequel le détecteur de rayons X (3) et la surface de support (9) du sein (100) sont mobiles le long de ladite première direction (Z).

8. Appareil (1) selon l'une quelconque des revendications précédentes, comprenant un actionneur (40), servant à déplacer le dispositif d'immobilisation (4), configuré pour déplacer le dispositif d'immobilisation (4) le long de la première direction (Z), l'actionneur (40) servant à déplacer le dispositif d'immobilisation (4) comprenant un mécanisme d'accouplement et de libération (41), mobile entre une position de couplage et une position de libération, configuré pour établir sélectivement un mode de déplacement automatique du dispositif d'immobilisation (4) le long de la première direction (Z), dans la position de couplage, ou pour déplacer manuellement le dispositif d'immobilisation (4) le long de la première direction (Z), dans la position de libération.

9. Appareil (1) selon l'une quelconque des revendications précédentes, dans lequel le dispositif d'immobilisation (4) comprend au moins un capteur de détection (42) configuré pour détecter la présence du sein (100) ou d'une partie du corps de la patiente.

10. Appareil (1) selon l'une quelconque des revendications précédentes, dans lequel le dispositif d'immobilisation (4) comprend au moins un premier capteur de force (43) et un second capteur de force (44), le premier capteur de force (43) étant configuré pour détecter un contact avec le sein (100) et le second capteur de force (44) étant configuré pour détecter une force appliquée sur le dispositif d'immobilisation (4).

11. Appareil (1) selon l'une quelconque des revendications précédentes, dans lequel le dispositif d'immobilisation (4) est mobile en rotation autour d'un deuxième axe de rotation (A2) parallèle au plan de détection (S).

12. Appareil (1) selon l'une quelconque des revendications précédentes, comprenant une pluralité de capteurs de surveillance (50) configurés pour détecter la présence d'un opérateur (200) et/ou de la patiente et/ou d'un composant de l'appareil (1) dans une zone de surveillance prédéterminée.

13. Appareil (1) selon l'une quelconque des revendications précédentes, comprenant un système de visualisation (60) configuré pour capturer des images du dispositif d'immobilisation (4) et/ou de la surface de support (9) du sein (100) et/ou du sein (100) et/ou de la patiente et/ou de l'opérateur (200), pour identifier la position ou les caractéristiques physiques d'un ou plusieurs desdits éléments parmi le dispositif d'immobilisation (4), la surface de support (9) du sein (100), le sein (100), la patiente, l'opérateur (200), par rapport à un système de référence prédéterminé.

14. Appareil (1) selon l'une quelconque des revendications précédentes, dans lequel le cadre de support (5), le premier actionneur (7A) et le second actionneur (7B) peuvent être inclinés, par rapport au cadre de base (6), au moyen d'une rotation autour d'un troisième axe de rotation horizontal (A3) entre une première position de fonctionnement (P3) et une seconde position de fonctionnement (P4) pour permettre la réalisation d'un examen diagnostique dans la première position de fonctionnement (P3) ou dans la seconde position de fonctionnement (P4).
